# EUROPEAN PATENT SPECIFICATION

(11) **EP 4 203 935 B1**
(45) Date of publication and mention of the grant of the patent: **23.07.2025**
(21) Application number: 21769135.1
(22) Date of filing: 27.08.2021
(51) Int. Cl.: A61K 31/192, A61K 31/405, A61K 31/455, A61K 31/706, A61K 31/7088, A61K 39/00, A61P 3/04, A61P 3/10, A61P 9/00, A61P 9/04, A61P 9/10, A61P 9/12

(54) **SPERMIDINE FOR USE IN THE PREVENTION AND/OR ALLEVIATION OF ADVERSE EFFECTS CAUSED BY NICOTINAMIDE**
SPERMIDIN ZUR VERWENDUNG BEI DER VORBEUGUNG UND/ODER ABSCHWÄCHUNG VON NEBENWIRKUNGEN VON NICOTINAMID
SPERMIDINE POUR L'UTILISATION DANS LA PRÉVENTION ET/OU L'ATTÉNUATION DES EFFETS INDÉSIRABLES CAUSÉS PAR LA NICOTINAMIDE

(30) Priority: 28.08.2020 EP 20193298
(43) Date of publication of application: 05.07.2023
(73) Proprietor: TLL The Longevity Labs GmbH, 8020 Graz (AT)
(72) Inventor: SEDEJ, Simon, 8044 Graz (AT); ABDELLATIF, Mahmoud, 8020 Graz (AT); EISENBERG, Tobias, 8010 Graz (AT); MADEO, Frank, 8044 Graz (AT)
(74) Representative: Schwarz & Partner Patentanwälte GmbH
(86) International application number: PCT/EP2021/073779
(87) International publication number: WO 2022/043515

(56) References cited:
- EP-A1- 3 320 899
- EP-A1- 3 636 254
- WO-A1-2019/106123
- CN-A- 108 245 513
- QI ZHENGTANG ET AL: "Long-term treatment with nicotinamide induces glucose intolerance and skeletal muscle lipotoxicity in normal chow-fed mice: compared to diet-induced obesity", THE JOURNAL OF NUTRITIONAL BIOCHEMISTRY, ELSEVIER, AMSTERDAM, NL, vol. 36, 4 August 2016 (2016-08-04), pages 31 - 41, XP029755641, ISSN: 0955-2863, DOI: 10.1016/J.JNUTBIO.2016.07.005
- TONG DAN ET AL: "Abstract 867: NAD+ Repletion Reverses HFpEF by Attenuating Myocardial Metabolic Dysfunction | Circulation Research", 16 October 2019 (2019-10-16), XP055871403, Retrieved from the Internet <URL:https://www.ahajournals.org/doi/10.1161/res.125.suppl_1.867> [retrieved on 20211209]
- ABDELLATIF MAHMOUD ET AL: "Nicotinamide for the treatment of heartfailure with preservedejection fraction", vol. 13, no. 580, 10 February 2021 (2021-02-10), pages ArticleNo, XP009531786, ISSN: 1946-6234, Retrieved from the Internet <URL:https://syndication.highwire.org/content/doi/10.1126/scitranslmed.abd7064> DOI: 10.1126/SCITRANSLMED.ABD7064

## Description

### TECHNICAL FIELD

The present invention relates to compositions for use in the prevention or alleviation of adverse effects on the glucose and/or insulin homeostasis caused by nicotinamide and/or a pharmaceutically acceptable salt thereof.

### BACKGROUND OF THE INVENTION

Cardiovascular diseases are the leading cause of morbidity and mortality worldwide. This is primarily due to a rapidly growing aged population and an ever-increasing obesity prevalence.

Cardiometabolic decline associated with aging, obesity and other contemporary risk factors (e.g. reduced physical activity and increased salt, calorie and processed food intake) manifest in the form of various cardiovascular diseases such as hypertension, atherosclerosis, coronary artery/ischemic heart disease, systolic and diastolic heart failure. These cardiovascular diseases cause reduced quality of life and increased premature disability, frailty, and mortality. Hence, safe, efficient, and feasible interventions with minimal or, if possible, no side effects that can directly target the underlying etiology of such cardiovascular health deterioration are urgently needed.

Many age- and obesity-related cardiovascular diseases either completely lack evidence-based therapy or rely on interventions that alleviate the symptoms or, at best, delay the disease progression and its adverse outcomes. One severe example is heart failure with preserved ejection fraction (HFpEF), also termed diastolic heart failure, arising from e.g. diastolic dysfunction. Diastolic heart failure accounts for about 50% of all heart failure cases in elderly but completely lacks medical treatment.

Individuals at high risk of cardiovascular complications are typically old and/or obese and suffer from hypertension and/or diabetes mellitus/metabolic syndrome. Therefore, these patients need a treatment to control the risk factors and reduce associated life-threatening events, such as heart attack, stroke, and heart failure.

Generally, the standard care in patients at cardiovascular risk consists of lipid-modifying compounds like statins and/or acetylsalicylic acid. Even with intensive statin and acetylsalicylic acid therapy, many patients remain at residual risk of cardiovascular complications. In addition, some patients are intolerant to these widely used pharmaceuticals, not to mention their controversial outcomes on primary disease prevention and their serious adverse effects, such as higher incidence of new-onset Diabetes Mellitus and myopathy (in case of statins) and major hemorrhagic events (with acetylsalicylic acid).

Sukhodub et al. (Pharmacol Res. 2010, 61(6): 564-570) describes the protection of the heart against ischaemia-reperfusion in mice by a nicotinamide-rich diet.

However, Reith and Armitage (Atherosclerosis 245(2016): 161-170) reviews different studies on the use of niacin and nicotinamide concluding that their administration may be associated with serious side effects on the glucose control and may be linked to an increase in new-onset of diabetes along with excesses of serious infections and bleeding (see also Zhengtang et al. (J Nutr Biochem. 2016, 36: 31-41)).

Spermidine and spermidine extracts can be used for the treatment of obesity-related disorders like type 2 diabetes and cardiovascular diseases. Further, it has been shown that spermidine may improve glucose tolerance and enhance insulin sensitivity (see WO 2019/106123 A1 and EP 3 320 899 A1).

EP 3 636 254 A1 discloses antioxidant compositions comprising spermidine, nicotinamide and at least one further component for the treatment of different oxidative stress diseases.

Thus, novel therapies of cardiovascular diseases having in particular fewer adverse effects are still of urgent need.

Hence, it is an object of the present invention to provide compositions for the prevention and/or treatment of cardiovascular diseases with decreased adverse effects compared to state-of-the-art compositions.

### SUMMARY OF THE INVENTION

The present invention relates to a composition comprising spermidine and/or a pharmaceutically acceptable salt thereof for use in the prevention and/or alleviation of adverse effects on the glucose and/or insulin homeostasis caused by nicotinamide and/or a pharmaceutically acceptable salt thereof. The invention is set out in the appended set of claims.

It has surprisingly been found that compositions comprising spermidine and/or a pharmaceutically acceptable salt thereof can be used in the prevention and/or alleviation of adverse effects caused by the lipid-modifying compound nicotinamide. Such adverse effects include for example adverse effects on the glucose and/or insulin homeostasis, such as glucose intolerance and obesity. Without being bound to a theory, the administration of a lipid-modifying compound, in particular nicotinamide, may lead to a decrease of the body's own spermidine, thus initiating adverse effects on the glucose and/or insulin homeostasis. Hence, in a first aspect of the present invention, spermidine and/or a pharmaceutically acceptable salt thereof can be used in the prevention and/or alleviation of adverse effects on the glucose and/or insulin homeostasis caused by nicotinamide and/or a pharmaceutically acceptable salt thereof.

Disclosed is also the use of nicotinamide, nicotinic acid and/or a pharmaceutically acceptable salt thereof, in the prevention and/or treatment of diastolic heart failure. It has been shown that nicotinamide, nicotinic acid and/or a pharmaceutically acceptable salt thereof is capable of replenishing myocardial nicotinamide and NAD⁺ levels, which in turn, may facilitate cardiovascular protection. Furthermore, it turned out that this beneficial effect was not mitigated by supplemental administration of spermidine.

Further disclosed is a pharmaceutical or food supplement composition comprising spermidine and/or a pharmaceutically acceptable salt thereof and nicotinamide, nicotinic acid and/or a pharmaceutically acceptable salt thereof, wherein the composition comprises spermidine and/or a pharmaceutically acceptable salt thereof and nicotinamide, nicotinic acid and/or a pharmaceutically acceptable salt thereof in a molar ratio ranging from 1:5 to 1:100. The combined administration of spermidine and/or a pharmaceutically acceptable salt thereof and nicotinamide, nicotinic acid and/or a pharmaceutically acceptable salt thereof has positive effects on patients suffering from cardiovascular diseases (being based on the at least one lipid-modifying compound), with reduced adverse effects compared to state of the art treatments (being based on the spermidine and/or a pharmaceutically acceptable salt thereof).

### SHORT DESCRIPTION OF THE FIGURES

Fig. 1 shows that nicotinamide and nicotinamide + spermidine increase myocardial nicotinamide and myocardial NAD⁺ levels (cardiometabolic syndrome model).
Fig. 2a shows that nicotinamide and nicotinamide + spermidine improve diastolic function (aging model).
Fig. 2b shows that nicotinamide improves diastolic function (salt-induced hypertensive heart disease model).
Fig. 2c shows that nicotinamide and nicotinamide + spermidine improve diastolic function (cardiometabolic syndrome model).
Fig. 3a shows that that nicotinamide and nicotinamide + spermidine improve cardiac hypertrophy (aging model).
Fig. 3b shows that that nicotinamide improves cardiac hypertrophy (salt-induced hypertensive heart disease model) .
Fig. 3c shows that that nicotinamide and nicotinamide + spermidine improve cardiac hypertrophy (cardiometabolic syndrome model).
Fig. 4a shows that nicotinamide improves exercise capacity (salt-induced hypertensive heart disease model).
Fig. 4b shows that nicotinamide and nicotinamide + spermidine improve exercise capacity (cardiometabolic syndrome model) .
Fig. 5a shows that nicotinamide reduces lung congestion (salt-induced hypertensive heart disease model).
Fig. 5b shows that nicotinamide and nicotinamide + spermidine reduce lung congestion (cardiometabolic syndrome model).
Fig. 6a shows that spermidine protects against side effects (i.e. glucose intolerance) of nicotinamide (fasting glucose in cardiometabolic syndrome model).
Fig. 6b shows that spermidine protects against side effects of nicotinamide (fasting insulin in cardiometabolic syndrome model).
Fig. 7 shows that spermidine prevents glucose intolerance induced by nicotinamide (cardiometabolic syndrome model).
Fig. 8 shows that spermidine alone has no positive impact on glucose intolerance in obese rats.

### DESCRIPTION OF THE EMBODIMENTS

"Spermidine", as used herein, is a biogenic polyamine from which spermine can be derived. Spermidine is present in most living organisms and plays a crucial role in growth.

Spermidine for use in this invention can be used in the form of its pharmaceutically acceptable salts. The salts include, for example, salts with inorganic acids such as hydrochloric acid, sulfuric acid, phosphoric acid and boric acid, and salts with organic acids such as acetic acid, citric acid, tartaric acid and glutaric acid. Hence, the term "pharmaceutically acceptable salt" of spermidine refers to one or more of the aforementioned salts, but is not limited thereto and includes also other possible salts of spermidine.

Nicotinamide and nicotinic acid are considered as "lipid-modifying compounds". As used herein, this term refers to compounds capable of modifying the level of a variety of lipids, as e.g. the level of low density lipoprotein (LDL) and high density lipoprotein (HDL), within an individual and mammal. An imbalance of lipid levels is associated with different diseases, as e.g. atherosclerosis, leading to cardiovascular diseases. Thus, lipid-modifying compounds are used to treat different lipid-associated diseases. The mode of action of these lipid-modifying compounds is diverse and tackles different steps of the lipid synthesis, uptake or metabolism.

"Prevention" or "preventing", as used herein, refers to the reduction of risk of a condition, disorder and/or disease. Ideally, the prevention of a condition, disorder and/or disease may result in a complete avoidance of a condition, disorder and/or disease in an individual or mammal. Both terms include also the avoidance of adverse effects caused by compounds administered to an individual or mammal.

"Alleviation" or "alleviating", as used herein, refers to the reduction of symptoms of a condition, disorder and/or disease or to the reduction of adverse effects caused by a compound.

The terms "treatment" and "treating", as used herein, refers to therapeutic measures to individuals or mammals, in order slow down or even stop the development of a path-ologic condition, disorder and/or disease and to ideally cure individuals or mammals, from a condition, disorder and/or diseases. The terms "treatment" and "treating", do not necessarily imply that an individual or mammal, is treated until total recovery.

The composition of the present invention can be administered to human individuals or to mammals, whereby mammals preferably include dogs, cats, horses and camels.

As discussed above, lipid-modifying compounds such as nicotinamide are known to have adverse effects when administered to individuals and mammals. These adverse effects have been mostly studied for other lipid-modifying compounds like statins. Spermidine and/or pharmaceutically acceptable salts thereof are able to alleviate these adverse effects, so that the lipid-modifying compounds, in particular nicotinamide, nicotinic acid and/or pharmaceutically acceptable salts thereof, can be safely administered to individuals and mammals. It turned out that spermidine and/or pharmaceutically acceptable salts thereof are particularly suited to be administered in combination with nicotinamide, nicotinic acid and/or pharmaceutically acceptable salts thereof.

Adverse effects caused by nicotinamide, nicotinic acid and/or pharmaceutically acceptable salts thereof can surprisingly be prevented and/or alleviated when administered before, together or consecutively to spermidine and/or a pharmaceutically acceptable salt thereof.

Spermidine and/or pharmaceutically acceptable salts thereof can be obtained from several sources. Hence, it is particularly preferred that these compounds are of natural, biotechnological and/or synthetical origin.

Besides the presence of spermidine in the mammalian body, plants and microorganisms produce spermidine as well. Therefore, plants and microorganisms (e.g. yeasts) represent a natural source of spermidine and pharmaceutically acceptable salts thereof.

In a particular preferred embodiment of the present invention spermidine and/or a pharmaceutically acceptable salt thereof of natural origin is provided as an extract, preferably as a plant extract, more preferably as a wheat germ extract or a soy bean extract. Wheat germs and soy beans are particular preferred as they are available in large amounts, thus being cheap, and comprise naturally high amounts of polyamines, in particular spermidine.

Plant extracts comprising spermidine can be obtained by methods known in the art (see e.g. EP19195898.2 or US 2014/378708).

Spermidine and/or pharmaceutically acceptable salts thereof of biotechnological origin, can be obtained in well-known expression systems via recombinant expression of the enzymes involved in the synthesis of spermidine, including spermidine synthase (converting putrescine to spermidine).

One way of synthesizing spermidine and thus obtaining spermidine of synthetic origin is described, for instance, in Jackson EL (J. Org. Chem. 21(1956):1374-1375).

As indicated above, lipid-modifying compounds like nicotinamide and nicotinic acid cause several adverse effects. It has been shown that nicotinamide leads to an increase in fasting blood glucose level and in fasting blood insulin level and thus facilitating a decrease in glucose tolerance, diabetes, in particular diabetes type 2, and obesity. Such adverse effects are also known to occur upon the treatment with statins, another group of known lipid-modifying compounds.

Thus, according to a preferred embodiment of the present invention, the adverse effect caused by nicotinamide include adverse effects on the glucose and/or insulin homeostasis, which are preferably selected from the group consisting of an increase in blood glucose level, in particular fasting blood glucose level, a decrease in glucose tolerance, obesity, diabetes, in particular diabetes type 2, and an increase in blood insulin level, in particular fasting blood insulin level.

The data of the present patent application impressively demonstrate that spermidine is able to reduce the fasting blood glucose level and the fasting blood insulin level and thus increase the glucose tolerance in a cardiometabolic syndrome model to levels comparable with the control group, which was not receiving any nicotinamide treatment. It has been surprisingly found that spermidine alone has no significant influence on fasting blood glucose level and the fasting blood insulin level. This surprising fact clearly shows that spermidine reduces specifically the adverse effects caused by nicotinamide. Therefore, it is shown that spermidine is effective and can thus be used in the prevention and/or alleviation of adverse effects of lipid-modifying compounds, as e.g. nicotinamide.

In a preferred embodiment, the composition of the present invention is administered before, together or consecutively to nicotinamide and/or pharmaceutically acceptable salts thereof. However, in the most preferred embodiment of the present invention the composition comprising spermidine and/or a pharmaceutically acceptable salt thereof is preferably administered together with nicotinamide and/or a pharmaceutically acceptable salt thereof (ideally in a single dosage form).

The composition comprising spermidine and/or a pharmaceutically acceptable salt thereof is preferably administered at a maximum of 6 hours, preferably at a maximum of 5 hours, more preferably at a maximum of 4 hours, more preferably at a maximum of 3 hours, more preferably at a maximum of 2 hours, more preferably at a maximum of 1 hour, more preferably at a maximum of 0.5 hours, more preferably at a maximum of 0.25 hours, or 0.25 to 6 hours, preferably 0.25 to 5 hours, more preferably 0.5 to 4 hours, more preferably 0.5 to 3 hours, more preferably 1 to 2 hours, in particular 1 hour, before or after the nicotinamide and/or a pharmaceutically acceptable salt thereof.

In a particular preferred embodiment of the present invention, the composition comprising spermidine and/or a pharmaceutically acceptable salt thereof is administered together with the nicotinamide and/or a pharmaceutically acceptable salt thereof. As demonstrated in the examples of the present patent application, this mode of administration results in an impressive prevention/alleviation of the adverse effects on the glucose and/or insulin homeostasis of nicotinamide, being a lipid-modifying compound.

As the concomitant administration led to such positive results, the composition comprises the nicotinamide and/or a pharmaceutically acceptable salt thereof in a further preferred embodiment. This has the further advantage, that both, the spermidine and/or a pharmaceutically acceptable salt thereof and the nicotinamide and/or a pharmaceutically acceptable salt thereof can be comprised in one single dosage form, by way of which the handling for the patient will be eased.

In order to prevent and/or alleviate the adverse effects on the glucose and/or insulin homeostasis caused by nicotinamide and/or pharmaceutically acceptable salts thereof, spermidine and/or a pharmaceutically acceptable salt thereof are provided and administered to an individual, preferably a mammal in a therapeutically effective amount. The term "therapeutically effective amount", as used herein, means that amount of a compound or combination that will elicit the desired biological or medical response in a mammal, preferably human subject.

The dose of spermidine and/or a pharmaceutically acceptable salt thereof administered can depend on various factors, as e.g. the constitution of the individual or mammal, or the severity of the adverse effects to be prevented and/or alleviated.

The spermidine and/or a pharmaceutically acceptable salt thereof are administered preferably in an amount of 0.001 to 5 mg/kg body weight per day, preferably 0.02 to 2 mg/kg body weight per day, more preferably 0.05 to 0.5 mg/kg body weight per day, more preferably 0.05 to 0.1 mg/kg body weight per day, in particular 0.08 mg/kg body weight per day.

Furthermore, the dose of administration of the nicotinamide and/or a pharmaceutically acceptable salt thereof is also subjected to variation, being as well dependent from several factors as e.g. the severity of the condition against which the nicotinamide and/or a pharmaceutically acceptable salt thereof is administered or the constitution of the individual or mammal. However, it is known that nicotinamide, for instance, is well-tolerated at high doses during chronical administration (Gale et al. (2004), The Lancet 363: 925-931; Chen et al. (2015), The New England Journal of Medicine 373:1618-1626; Poyan et al. (2018), Nature Medicine 24(9): 1351-1359).

Thus, nicotinamide and/or a pharmaceutically acceptable salt thereof is preferably administered in an amount of 0.01 to 25 mg/kg body weight per day, preferably 0.1 to 20 mg/kg body weight per day, more preferably 0.1 to 15 mg/kg body weight per day, more preferably 0.1 to 12.5 mg/kg body weight per day, more preferably 0.2 to 10 mg/kg body weight per day, more preferably 0.3 to 8 mg/kg body weight per day, more preferably 0.5 to 6 mg/kg body weight per day, in particular 1 to 5 mg/kg body weight per day.

A certain ratio of spermidine and/or a pharmaceutically acceptable salt thereof to the nicotinamide and/or a pharmaceutically acceptable salt thereof to be administered per day is advantageous.

According to a preferred embodiment of the present invention, the spermidine and/or a pharmaceutically acceptable salt thereof and the nicotinamide and/or pharmaceutically acceptable salt thereof are administered in a molar ratio ranging from 1:5 to 1:100, preferably 1:5 to 1:75, more preferably 1:5 to 1:50, more preferably from 1:8 to 1:25, more preferably 1:10 to 1:15.

It is particularly preferred, that the composition comprising nicotinamide and/or a pharmaceutically acceptable salt thereof as well is combined with the preferred embodiment of molar ratios. In this way an effective amount of both, the spermidine and/or a pharmaceutically acceptable salt thereof and the nicotinamide and/or pharmaceutically acceptable salt thereof, can be set and the doses of both, the spermidine and/or a pharmaceutically acceptable salt thereof and the nicotinamide and/or pharmaceutically acceptable salt thereof, can be adapted to each other, depending on the intended use, to facilitate the maximum possible effect of both. This increases the effectiveness of the prevention/alleviation/treatment and in addition eases the handling, especially for the individual or mammal, in need.

The composition of and/or used according to the present invention can be administered to an individual or mammal, by various routes. Preferred administration routes include oral and intravenous administration, whereby oral administration is particularly preferred.

The dosage forms used to administer the composition of and/or used according to the present invention are preferably adapted to the route of administration.

The composition may be formulated in a capsule, as a tablet, as suspension, as solution, as powder or in a gel.

The composition used according to the present invention may comprise next to spermidine and/or pharmaceutically acceptable salts thereof and/or the nicotinamide and/or pharmaceutically acceptable salt thereof various excipients depending on the route of administration. The use of excipients for pharmaceutically active substances is well known in the art.

Tablets of the present invention may be made by compression or molding, optionally with one or more additional ingredients. Compressed tablets may be prepared using a binder (for example, gelatin or hydroxypropylmethyl cellulose), a lubricant, an inert diluent, a preservative, a disintegrant (for example, sodium starch glycolate or cross-linked sodium carboxymethyl cellulose), a surface-active or a dispersing agent. Molded tablets may be made by molding in a suitable machine a mixture of a powdered spermidine and/or a pharmaceutically acceptable salt thereof comprising composition moistened with an inert liquid diluent.

The tablets, and other solid dosage forms of the pharmaceutical compositions of and used according to the present invention, such as dragees, capsules, pills and granules, may optionally be prepared with coatings, such as enteric coatings and other coatings well known in the art. They may also be formulated so as to provide slow or controlled release of the active ingredient therein using, for example, hydroxypropylmethyl cellulose in varying proportions to provide the desired release profile, other polymer matrices, liposomes or microspheres.

Spermidine and/or pharmaceutically acceptable salts thereof and/or the nicotinamide and/or pharmaceutically acceptable salt thereof may be formulated to release the active ingredient only, or preferentially, in a certain portion of the gastrointestinal tract, optionally, in a delayed manner. Examples of embedding compositions which may be used include polymeric substances and waxes. The active ingredient may also be in micro-encapsulated form, if appropriate, with one or more of the above-described excipients.

Liquid dosage forms for oral administration of the compounds of and/or used according to the present invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitol, and mixtures thereof. Besides inert diluents, the oral compositions may also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and mixtures thereof.

Disclosed is also a composition comprising nicotinamide, nicotinic acid and/or a pharmaceutically acceptable salt thereof for use in the prevention and/or treatment of diastolic heart failure.

As shown by the appended inventive examples, nicotinamide is capable of preventing/treating various cardiovascular diseases, as e.g. hypertensive heart disease or diastolic heart failure.

Depending on if and which additional compounds may be used for the prevention/treatment of the cardiovascular disease, the composition may comprise differing amounts of nicotinamide, nicotinic acid and/or pharmaceutically acceptable salts thereof.

One example of a compound used in addition to the nicotinamide, nicotinic acid and/or pharmaceutically acceptable salt thereof may be spermidine and/or a pharmaceutically acceptable salt thereof, which has the capability to prevent and/or alleviate adverse effects caused by nicotinamide and/or pharmaceutically acceptable salts thereof. As surprisingly demonstrated by the appended data, nicotinamide exerted its preventive/treating effect on diastolic heart failure even at the combined administration with spermidine.

The composition comprises spermidine and/or a pharmaceutically acceptable salt thereof or can be administered before, together or consecutively to spermidine and/or a pharmaceutically acceptable salt thereof.

The composition comprising spermidine and/or a pharmaceutically acceptable salt thereof is preferably administered at a maximum of 6 hours, preferably at a maximum of 5 hours, more preferably at a maximum of 4 hours, more preferably at a maximum of 3 hours, more preferably at a maximum of 2 hours, more preferably at a maximum of 1 hour, more preferably at a maximum of 0.5 hours, more preferably at a maximum of 0.25 hours, or 0.25 to 6 hours, preferably 0.25 to 5 hours, more preferably 0.5 to 4 hours, more preferably 0.5 to 3 hours, more preferably 1 to 2 hours, in particular 1 hour, before or after nicotinamide, nicotinic acid and/or a pharmaceutically acceptable salt thereof.

The composition comprising spermidine and/or a pharmaceutically acceptable salt thereof can be administered together with nicotinamide, nicotinic acid and/or a pharmaceutically acceptable salt thereof. The combined administration has proven its effectivity in preventing/treating cardiovascular diseases, as e.g. diastolic heart failure.

Thus, it is particularly preferred, that the composition comprises in addition to nicotinamide, nicotinic acid and/or a pharmaceutically acceptable salt thereof spermidine and/or a pharmaceutically acceptable salt thereof. This way the handling for the individual or mammal, will be eased, as the composition comprising both, the spermidine and/or a pharmaceutically acceptable salt thereof and nicotinamide, nicotinic acid and/or a pharmaceutically acceptable salt thereof can thus be comprised e.g. in one single dosage form.

As outlined above, the composition preferably comprises mainly nicotinamide, nicotinic acid and/or a pharmaceutically acceptable salt thereof. Thus, spermidine and/or a pharmaceutically acceptable salt thereof is comprised in a much smaller amount, depending on e.g. the constitution of the individual or mammal, and the reason for its administration.

The spermidine and/or a pharmaceutically acceptable salt thereof is of natural origin, biotechnological origin and/or synthetic origin.

As outlined above, spermidine and/or a pharmaceutically acceptable salt thereof can be produced by e.g. the human body and by plants. Therefore, plants represent a natural source of spermidine.

The spermidine and/or a pharmaceutically acceptable salt thereof of natural origin can be provided as an extract, preferably as a plant extract, more preferably as a wheat germ extract or a soy bean extract.

Also preferred is spermidine and/or a pharmaceutically acceptable salt thereof of synthetic origin.

As known from literature (Gale et al. (2004), The Lancet 363: 925-931; Chen et al. (2015), The New England Journal of Medicine 373:1618-1626; Poyan et al. (2018), Nature Medicine 24(9): 1351-1359), nicotinamide is well-tolerated at high doses during chronical administration. Thus, an administration of high doses is possible. However, the dose should be adjusted based on the condition of the individual or human, and the particular cardiovascular disease to be prevented and/or treated.

Nicotinamide, nicotinic acid and/or a pharmaceutically acceptable salt thereof can be administered in an amount of 0.01 to 25 mg/kg body weight per day, preferably 0.1 to 20 mg/kg body weight per day, more preferably 0.1 to 15 mg/kg body weight per day, more preferably 0.1 to 12.5 mg/kg body weight per day, more preferably 0.2 to 10 mg/kg body weight per day, more preferably 0.3 to 8 mg/kg body weight per day, more preferably 0.5 to 6 mg/kg body weight per day, in particular 1 to 5 mg/kg body weight per day.

Spermidine and/or a pharmaceutically acceptable salt thereof may be administered in a lower daily dose than the at least one nicotinamide adenine dinucleotide precursor. Again, the amount may depend on the condition of the individual or mammal, but also on the amount of nicotinamide, nicotinic acid and/or pharmaceutically acceptable salts thereof administered to ensure its activity against cardiovascular diseases.

Spermidine and/or a pharmaceutically acceptable salt thereof can be administered in an amount of 0.001 to 5 mg/kg body weight per day, preferably 0.02 to 2 mg/kg body weight per day, more preferably 0.05 to 0.5 mg/kg body weight per day, more preferably 0.05 to 0.1 mg/kg body weight per day, in particular 0.08 mg/kg body weight per day.

A specific ratio of administered nicotinamide, nicotinic acid and/or pharmaceutically acceptable salt thereof and spermidine and/or a pharmaceutically acceptable salt thereof is preferred, as by way of such the effective amount nicotinamide, nicotinic acid and/or pharmaceutically acceptable salt thereof can be combined with an amount of spermidine and/or a pharmaceutically acceptable salt thereof not interfering with the effect of nicotinamide, nicotinic acid and/or pharmaceutically acceptable salt thereof, but still exerting its function.

Thus, the spermidine and/or a pharmaceutically acceptable salt thereof and nicotinamide, nicotinic acid and/or a pharmaceutically acceptable salt thereof can be administered in a molar ratio ranging from 1:5 to 1:100, preferably 1:5 to 1:75, more preferably 1:5 to 1:50, more preferably from 1:8 to 1:25, more preferably 1:10 to 1:15.

The composition can be administered orally or intravenously. The oral administration of the composition is most preferred.

As outlined above and demonstrated in the inventive examples, the at least one nicotinamide adenine dinucleotide precursor, in particular nicotinamide, is effective in preventing/treating various cardiovascular diseases, e.g. via improving diastolic function, cardiac hypertrophy and exercise capacity, and via reducing lung congestion, being tested in at least two of the following model conditions: high ageing, salt-induced hypertensive heart disease and cardiometabolic syndrome.

According to the appended data, the effect of nicotinamide, nicotinic acid and/or a pharmaceutically acceptable salt thereof on cardiovascular diseases was even present at the combined administration with spermidine. In addition, however, spermidine reduced the adverse effect of nicotinamide on the glucose and/or insulin homeostasis. Thus, the administration of nicotinamide, being a particular preferred example of the at least one nicotinamide adenine dinucleotide precursor, with spermidine outperforms the standard therapy of cardiovascular disease regarding effectiveness with combined reduced adverse effects.

Further disclosed is a pharmaceutical or food supplement composition comprising spermidine and/or a pharmaceutically acceptable salt thereof and nicotinamide, nicotinic acid and/or a pharmaceutically acceptable salt thereof.

The composition comprises spermidine and/or a pharmaceutically acceptable salt thereof and nicotinamide, nicotinic acid and/or a pharmaceutically acceptable salt thereof in a molar ratio ranging from 1:5 to 1:100, preferably 1:5 to 1:75, more preferably 1:5 to 1:50, more preferably from 1:8 to 1:25, more preferably 1:10 to 1:15, in particular 1:13.

The spermidine and/or a pharmaceutically acceptable salt thereof is of natural origin, biotechnological origin and/or synthetical origin.

The spermidine and/or a pharmaceutically acceptable salt thereof of natural origin can be provided as an extract, preferably as a plant extract, more preferably as a wheat germ extract or a soy bean extract.

The present invention is further illustrated by the following examples, however, without being restricted thereto.

### EXAMPLES

### Experimental models, animal maintenance and treatment

Aging model: Aged C57BL/6J male mice were purchased from the Charles River Laboratories (Sulzfeld, Germany) at the age of 18 months and fed standard chow. Supplementation of 24 mM nicotinamide (i.e. 0.3% w/v, changed two times per week) and of spermidine (i.e. 3 mM final concentration at pH 7.2, changed two times per week) in the regular drinking water was conducted at 20 months of age for a period of 4 months. This nicotinamide concentration was calculated due to the interspe-cies metabolic rate variability (rat versus mouse), thereby considering average daily water consumption and animal equivalent dose calculation based on body surface area as reported previously (Nair and Jacob (2016), J Basic Clin Pharm 7(2): 27-31). Spermidine concentration was based on previous reports and preparation of stock solutions followed published protocols (e.g. Eisenberg et al. (2009), Nature Cell Biology 11: 1305-1314).

Salt-induced hypertensive heart disease model: Dahl salt-sensitive male rats (n=24) were purchased at the age of 4 weeks from the Charles River Laboratories (Kingston, USA). Eight rats were fed the low-salt diet containing 0.3% NaCl (AIN-76A, Research Diets, Inc. USA) throughout the experiment and served as a healthy control group. Low-salt diet was fed also to the remaining 16 Dahl rats until the age of 7 weeks, when the diet was switched to a high-salt diet (AIN-76A with 8% NaCl, Research Diets, Inc. USA), and the rats were divided into two weight-matched cohorts each consisting of two equally sized groups (n = 8 animals per group); group 1 received regular drinking water (non-treated control), while group 2 received regular drinking water supplemented with 40 mM nicotinamide (i.e. 0.5% w/v) for a period of 12 weeks.

Cardiometabolic syndrome model: ZSF1 lean and obese rats of both genders were purchased at the age of 5 weeks from the Charles River Laboratories (Kingston, USA). Eight-week-old ZSF1 obese rats were divided into three weight-matched and equally sized/gender-balanced groups: group 1 received regular drinking water (non-treated control), group 2 received regular drinking water supplemented with 40 mM nicotinamide (i.e. 0.5% w/v), and group 3 was administered the combination of 40 mM nicotinamide and 3 mM spermidine (of synthetical origin) in drinking water for a period of 14 weeks. Water as well as food intake were monitored regularly. Age-matched untreated ZSF1 obese and lean rats served as controls.

Animals were housed under conventional (rats) or specific-pathogen-free (SPF) (mice) conditions in a 12h light and 12h dark cycle with *ad libitum* access to water and food. Autoclaved nest material and red plastic houses served as cage enrichment for mice.

The experimenters were blinded to the treatment of the animals. Cardiometabolic phenotyping, including echocardiography-based assessment of cardiac function and exercise capacity coupled to indirect calorimetry were performed at the age of 21-22 weeks, whereas glucose tolerance testing was performed at 8 and 18 weeks of age.

### Example 1: Increase of myocardial nicotinamide and myocardial NAD⁺ level

Herein the effect of nicotinamide administration on myocardial nicotinamide and myocardial NAD⁺ levels was examined.

### Method:

The mass spectrometric metabolomic analysis of nicotinamide and NAD⁺ in rat myocardium was performed as described previously (Viltard M., et al. Aging (Albany NY) 11 (2019) :4783-4800) .

### Sample preparation:

About 30mg of tissue was weighed and solubilized in tubes with ceramic beads using 1ml of cold lysate buffer (methanol:water ratio was 9:1; at -20°C). Tissue samples were then homogenized three times for 20s at 5.500rpm using a Precellys 24 tissue homogenizer followed by centrifugation for 10min at 15.000g and 4°C.

300µl of the upper phase of the supernatant was used for the ultrahigh pressure liquid chromatography coupled with mass spectrometry (UHPLC-MS), evaporated at 40°C in a pneumatically assisted concentrator. Dried extract were then solubilized with 200µl of deionized water, aliquoted or transferred in LC vials and injected into UHPLC-MS or kept at -80°C until injection.

The GC-MS aliquots (300µl) were evaporated and dried, and 50µL of methoxyamine (20mg/ml in pyridine) was added, after which the aliquots were stored at room temperature in the dark for 16h. Next day, 80µl of MSTFA was added and final deri-vatization was performed at 40°C for 30min. Samples were then directly injected into GC-MS.

Measurement of NAD⁺ levels by ion pairing ultrahigh performance liquid chromatography (UHPLC) coupled to a Triple Quadrupole (QQQ) mass spectrometer:

Targeted analysis was performed on a RRLC 1260 system coupled to a Triple Quadrupole 6410 equipped with an electrospray source operating in positive mode. The gas temperature was set to 350°C with a gas flow of 12 liter/min. The capillary voltage was set to 3.5 kV. Ten microliters of sample was injected on a Column XDB-C18 (100 mm x 2.1 mm particle size 1.8 µm) from Agilent technologies, protected by a guard column XDB-C18 (5 mm × 2.1 mm particle size 1.8 µm) and heated at 40°C by a Pelletier oven. The gradient mobile phase consisted of water with 2 mM of DBAA (A) and acetonitrile (B). The flow rate was set to 0.2 mL/min, and gradient as follow: initial condition was 90% phase A and 10% phase B, maintained during 4 min. Molecules were then eluted using a gradient from 10% to 95% phase B over 3 min. The column was washed using 95% mobile phase B for 3 minutes and equilibrated using 10% mobile phase B for 3 min. The autosampler was kept at 4°C. The collision gas was nitrogen. The scan mode used was the MRM for biological samples. Peak detection and integration of the analytes were performed using the Agilent Mass Hunter quantitative software (B.07.01).

Measurement of nicotinamide levels by gas chromatography (GC) coupled to a triple quadrupole (QQQ) mass spectrometer:

GC-MS/MS method was performed on a 7890B gas chromatography coupled to a triple quadrupole 7000C equipped with a High sensitivity electronic impact source (EI) operating in positive mode.

### Results:

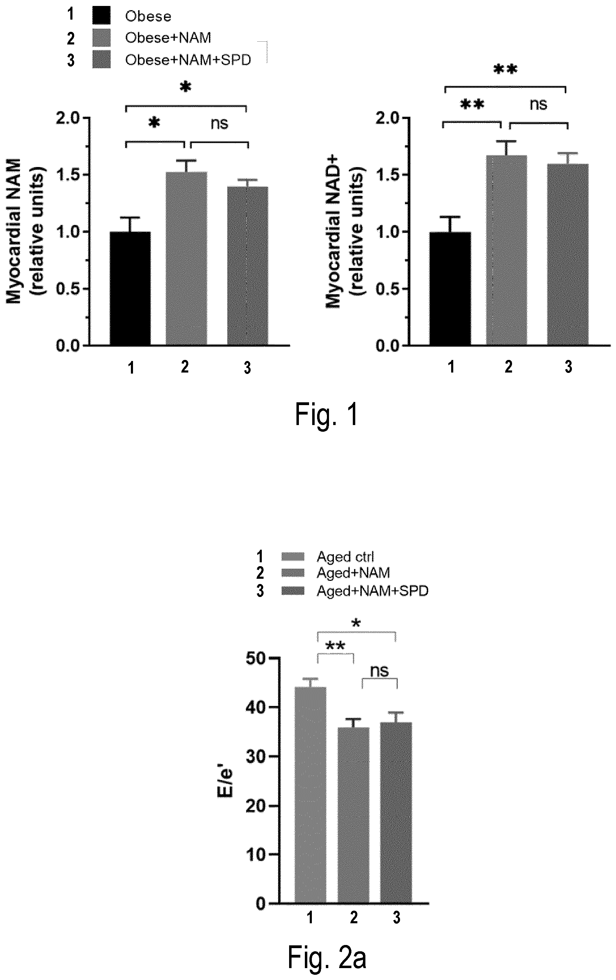

Fig. 1 shows that upon treatment with nicotinamide the myocardial level of nicotinamide (NAM) (Fig. 1, left) and nicotinamide adenine dinucleotide + (NAD⁺) (Fig. 1, right) significantly increased in a cardiometabolic syndrome model. This effect was not significantly reduced by the addition treatment with spermidine (NAM+SPD). Thus, the combined treatment of nicotinamide and spermidine led to a significant increase of myocardial levels of nicotinamide and NAD⁺.

### Example 2: Improvement of cardiovascular parameters by the treatment with nicotinamide:

Herein the positive treatment effect of nicotinamide on cardiovascular parameters as diastolic function (i.e. item 2.1), cardiac hypertrophy (i.e. item 2.2), exercise capacity (i.e. 2.3) and lung congestion (i.e. item 2.4) is described.

### 2.1 - Measurement of the diastolic function

### 2.1.1 - Measurement of the diastolic function in the aging model:

### Method (Echocardiography):

Transthoracic echocardiography was performed using high resolution micro-imaging system equipped with a 24- and 55-MHz linear array transducers for rats and mice, respectively, as described (Eisenberg et al. (2016), Nat Med 22(12): 1428-1438). Briefly, lightly anesthetized mice (0.5% isoflurane and 99.5% O₂) were placed in the supine position on a temperature-controlled heating platform to maintain their body temperature at 37 °C. Diastolic function was assessed using pulsed-wave and tissue Doppler imaging to determine the ratio between peak early-filling velocity of transmitral flow (E) and the corresponding mitral valve annulus velocity (e'), respectively.

### Results:

Fig. 2a shows that the quotient E/e' (being a measure of diastolic dysfunction) was reduced upon treatment with nicotinamide (Aged + NAM), suggesting improved diastolic function. Thus, nicotinamide led to an improvement of diastolic function in the aging model. This effect was also present when nicotinamide was administered with spermidine (24M + NAM + SPD).

### 2.1.2 - Measurement of the diastolic function in the salt-induced hypertensive heart disease model:

### Method (Hemodynamic pressure-volume measurement):

Rats were anesthetized by inhalation of isoflurane (5% for induction and 2-3% for maintenance), orotracheally intubated (14 G), and then mechanically ventilated (SAR-1000) to provide sufficient ventilation through controlling the respiration settings, while placed in a supine position on a temperature-controlled warming pad (TC-1000) to maintain body temperature at 37 °C. The heart rate was monitored by an electrocardiogram (Animal Bio Amp, FE136) and kept at 350-400 bpm throughout the experiment. Experiments were conducted in an open chest approach/method by performing thoracotomy to widely open the pericardium and expose the heart, and a pressure-volume catheter (SPR-847) was inserted along the ventricular long axis by apical puncture (24 G). Signals were continuously acquired (MPVS PL3508 PowerLab 8/35), digitized at 2 kHz (ML880 PowerLab 16/30), and analyzed offline (LabChart 8 Pro). After a stabilization period of 10 min, left ventricular end-diastolic pressure (EDP), a baseline parameter of diastolic function, was measured and averaged from ten consecutive beats, with ventilation suspended at end-expiration. After completion of experiments, animals were euthanized by exsanguination (blood collection) under deep anesthesia (5% isoflurane and 95% O₂) and organs were harvested, weighed and stored in liquid nitrogen for further tissue processing.

### Results:

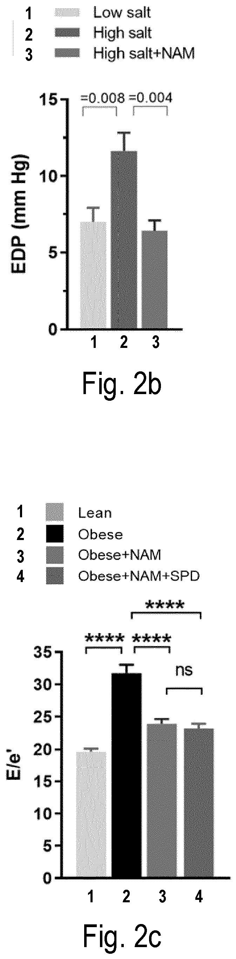

EDP is a marker for diastolic dysfunction, which was increased in the salt-induced_hypertensive heart disease model (high salt) and reduced upon treatment with nicotinamide (high salt + NAM). Thus, Fig. 2b shows that nicotinamide treatment improved diastolic function in the salt-induced hypertensive heart disease model.

### 2.1.3 - Measurement of the diastolic function in the cardiometabolic syndrome model:

### Method (Echocardiography):

This measurement was conducted according to the method of item 2.1.1, with the exception that the rats were lightly anesthetized with 1-2% isoflurane and 98-99% O₂.

### Results:

Fig. 2c shows that the quotient E/e' (being a measure of diastolic dysfunction) was increased in the cardiometabolic syndrome model (obese) and reduced upon treatment with nicotinamide (obese + NAM). Thus, the nicotinamide led to an improvement of diastolic function (reduced E/e') in the cardiometabolic syndrome model. This effect of nicotinamide treatment was also observed when administered with spermidine (obese + NAM + SPD).

### 2.2 - Measurement of the cardiac hypertrophy:

### 2.2.1 - Measurement of the cardiac hypertrophy in the aging model:

### Method (Gravimetry):

The heart was harvested and weighed, obtaining the heart weight (HW). For normalisation, the weight was subsequently divided by the length of the left tibia bone (TL), resulting in the ratio HW/TL.

### Results:

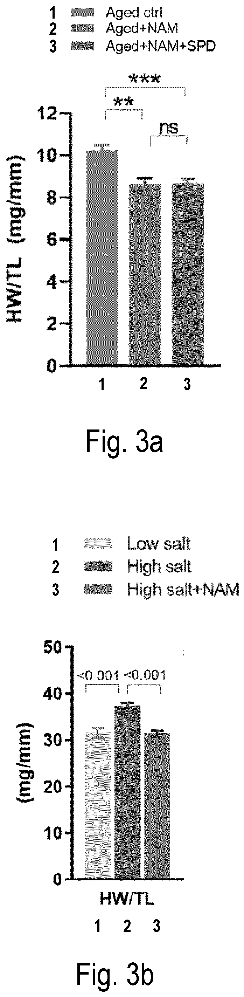

Fig. 3a shows that the heart weight-to-tibia length ratio (HW/TL), a marker of cardiac hypertrophy, was reduced upon treatment with nicotinamide (Aged + NAM) in the aging model. The same effect was observed upon combined administration of nicotinamide and spermidine (24 M + NAM + SPD).

### 2.2.2 - Measurement of the hypertrophy in the salt-induced hypertensive heart disease model:

### Method (Gravimetry):

This measurement was conducted according to the method of item 2.2.1.

### Results:

Fig. 3b shows that the heart weight-to-tibia length ratio (HW/TL), a marker of cardiac hypertrophy, was increased in the salt-induced hypertensive heart disease model (High salt). However, upon treatment with nicotinamide (high salt + NAM) the ratio HW/TL was reduced to levels similar to the control (Low salt).

### 2.2.2 - Measurement of the hypertrophy in the cardiometabolic syndrome model:

### Method (Gravimetry):

This measurement was conducted according to the method of item 2.2.1.

### Results:

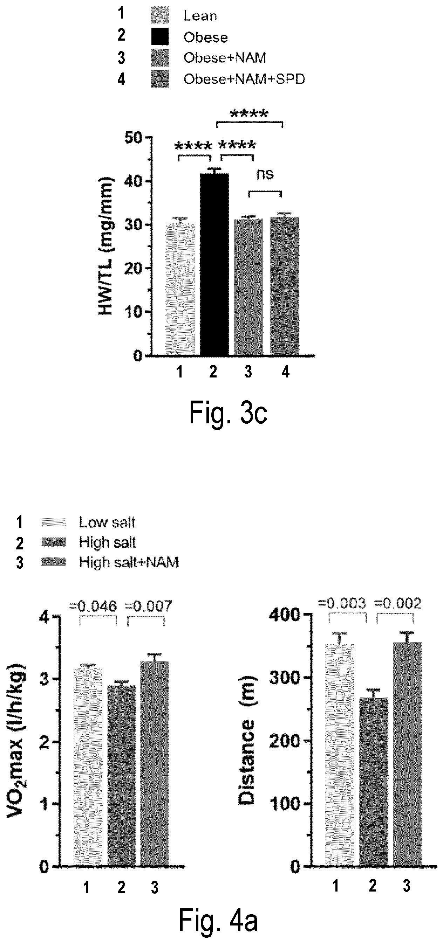

Fig. 3c shows that the heart weight-to-tibia length ratio (HW/TL), a marker of cardiac hypertrophy, was increased in the cardiometabolic syndrome model (Obese). However, the ratio was reduced upon treatment with nicotinamide (Obese + NAM) to levels similar to the ones of the control (Lean). The same effect was observed upon combined administration of nicotinamide and spermidine (Obese + NAM + SPD).

### 2.3 - Measurement of the exercise capacity:

### 2.3.1 - Measurement of the exercise capacity in the hypertensive heart disease model:

### Method (Exercise calorimetry):

Rats were subjected to peak effort on a closed-chamber treadmill at an inclination of 10° coupled to a gas analyzer (CaloTreadmill). After an initial adaption period of 3 min at 15 cm/s, testing velocity then continuously increased by 5 cm/s every minute to assess VO₂ max in peak effort protocol. The exercise session ended at the maximal fatigue, which was defined as the lack of ability to maintain running speed despite being in contact with the electric shock for more than 5 seconds. Calorimetric parameters, such as relative oxygen consumption (ΔVO₂) and carbon dioxide production (ΔVCO₂) were continuously measured at 0.2 Hz intervals during treadmill running. Maximal oxygen consumption (VO₂max) and run distance were determined at the point at which oxygen uptake reached a plateau during exhaustive workout/exercise.

### Results:

Fig. 4a shows that both, the VO₂max and the distance, decrease in the salt-induced hypertensive heart disease model (High salt). However, the nicotinamide treatment improved exercise capacity in the hypertensive heart disease model, as both the VO₂max and the distance were increased upon the treatment (High salt + NAM) to similar or even higher levels as in the controls (Low salt).

### 2.3.2 - Measurement of the exercise capacity in the cardiometabolic syndrome model:

### Method (Exercise calorimetry):

This measurement was conducted according to the method of item 1.2.1.

### Results:

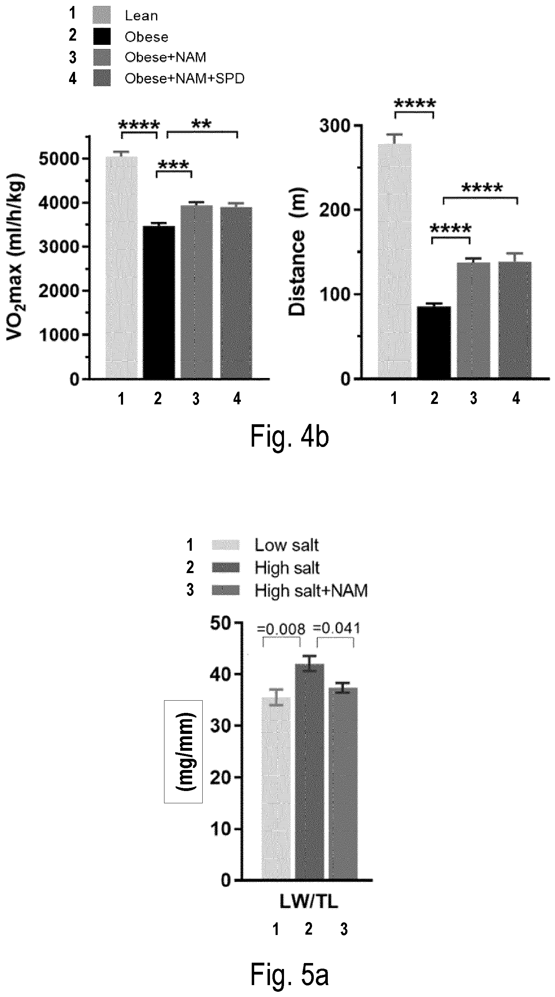

Fig. 4b shows that both the VO₂max and the distance decrease in the cardiometabolic syndrome model (Obese). However, the nicotinamide treatment improved exercise capacity in the cardiometabolic syndrome model, as both the VO₂max and the distance were increased upon the treatment (Obese + NAM). This effect was also present when nicotinamide was administered with spermidine (Obese + NAM + SPD).

### 2.4 - Measurement of the lung congestion:

### 2.4.1 - Measurement of the lung congestion in the hypertensive heart disease model:

### Method (Gravimetry):

The lung was harvested and weighed, obtaining the lung weight (LW). For normalisation, the weight was subsequently divided by the length of the left tibia bone (TL), resulting in the ratio LW/TL.

### Results:

Fig. 5a shows that the ratio LW/TL, being a measure of lung congestion, is increased in the salt-induced hypertensive heart disease model (High salt), indicating heart failure. However, nicotinamide treatment (High salt + NAM) led to a decrease of the ratio, implicating reduced lung congestion in the salt-induced hypertensive heart disease model.

### 2.4.2 - Measurement of the lung congestion in the cardiometabolic syndrome model:

### Method (Gravimetry):

This measurement was conducted according to the method of item 2.4.1.

### Results:

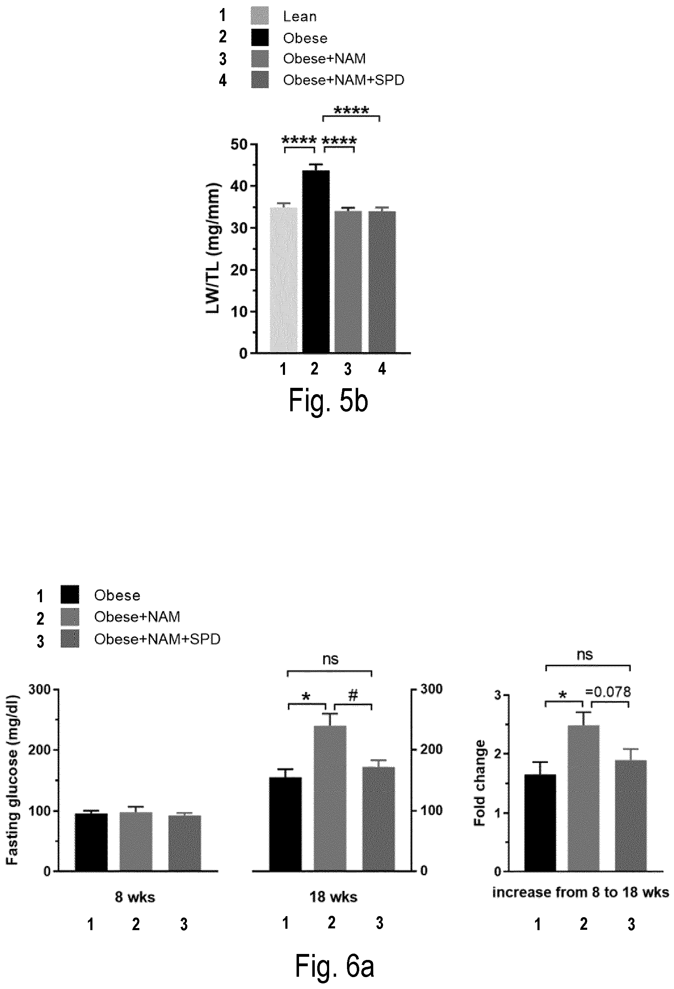

Fig. 5b shows that the ratio LW/TL, being a measure of lung congestion, is increased in the cardiometabolic syndrome model (Obese). However, nicotinamide treatment (Obese + NAM) compensated this effect, implicating reduced lung congestion in cardiometabolic syndrome model, this effect being also present at the combined administration of nicotinamide and spermidine (Obese + NAM + SPD).

### Summary:

Via replenishing myocardial NAD⁺ levels, nicotinamide improves several cardiovascular parameters, such as diastolic function, cardiac hypertrophy and exercise capacity, and leads to reduced lung congestion, being tested in at least two of the following model conditions: aging, salt-induced hypertensive heart disease and cardiometabolic syndrome. In addition it was shown that these effects of nicotinamide are not diminished by the combined administration of nicotinamide with spermidine.

### Example 3: Protective effect of spermidine against the side effects of nicotinamide:

Example 3 describes the protective effect of spermidine against the side effect of nicotinamide on the fasting glucose (i.e. item 3.1), on the fasting insulin (i.e. item 3.2) and on the glucose tolerance (i.e. item 3.3), being tested in the cardiometabolic syndrome model.

### 3.1 - Measurement of the fasting glucose:

### Method (Fasting glucose determination):

The basal fasting blood glucose level was determined by collecting a drop of blood from the tail vein. Blood droplet was applied to a test strip that was inserted into a glucose analyzer (glucometer, Accu-Chek Performa). The sampling and measurement was conducted at the age of 8 weeks (baseline) and at the age of 18 weeks. The fold-change was calculated by dividing the values form 18-weeks old animals and 8-weeks old animals.

### Results:

Fig. 6a shows a side effect of nicotinamide (Obese + NAM) treatment: the fasting glucose level is increased upon 10 weeks of nicotinamide treatment (8 weeks vs. 18 weeks), indicating glucose intolerance. However, this negative side effect of nicotinamide can be reduced (almost compensated) by spermidine (Obese + NAM + SPD).

### 3.2 - Measurement of the fasting insulin:

### Method (Fasting insulin determination):

Fasted blood samples were collected from the clavicular vena plexus/jugular vein of 6-h fasted (6AM-12AM) and lightly anesthetized rats (2% isoflurane and 98% O₂), whose body temperature was maintained at 37 °C using a temperature-controlled heating platform. Fasting insulin was measured in plasma using ultrasensitive rat ELISA kits (Crystal Chem) according to the manufacturer's protocol. The sampling and measurement was conducted at the age of 8 weeks (baseline) and at the age of 18 weeks. The fold-change was calculated by dividing the values form 18-weeks old animals and 8-weeks old animals.

### Results:

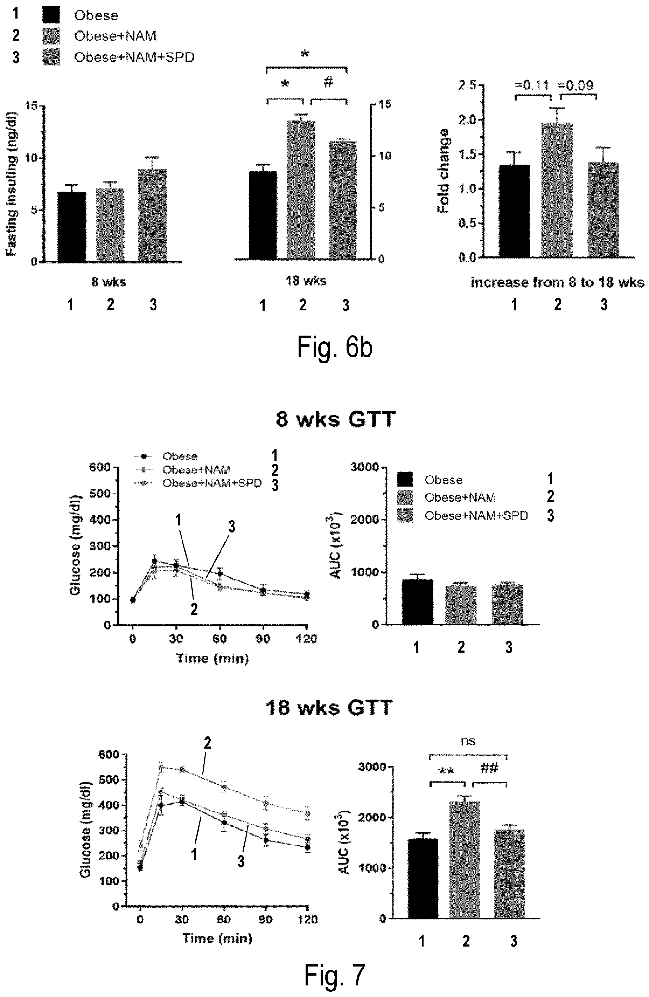

Fig. 6b shows another side effect of nicotinamide (Obese + NAM) treatment: the fasting insulin level is increased upon 10 weeks of nicotinamide treatment (8 weeks vs. 18 weeks). However, this negative side effect of nicotinamide can be compensated by spermidine (Obese + NAM + SPD).

### 3.3 - Measurement of the glucose tolerance:

### Method (Glucose tolerance test, GTT):

Intraperitoneal glucose tolerance test was performed at 6AM from 12-h fasted rats. During the experiment, rats were singly housed in empty cages without food, water or bedding. The basal fasting blood glucose level was determined by collecting a drop of blood from the tail vein. Blood droplet was applied to a test strip that was inserted into a glucose analyzer (Accu-Chek Performa). Rats were then injected intraperitoneally with 1 g of glucose per kg fasting body mass, and blood glucose levels were determined 15, 30, 60, 90 and 120 min after glucose. The sampling and measurement were conducted at the age of 8 weeks (baseline) and at the age of 18 weeks.

### Results:

Fig. 7 shows another side effect of nicotinamide (Obese + NAM) treatment: the induced glucose intolerance. It can be seen that upon 10 weeks of nicotinamide (Obese + NAM) treatment (8 weeks vs 18 weeks) the glucose level over time (represented as AUC as well) is higher compared to the control (Obese). However, this negative side effect of nicotinamide can be reduced by spermidine (Obese + NAM + SPD).

### 3.4 - Measurement of the glucose tolerance:

### Method

Intraperitoneal glucose tolerance test was performed at 6AM from 12-h fasted rats. During the experiment, rats were singly housed in empty cages without food, water or bedding. The basal fasting blood glucose level was determined by collecting a drop of blood from the tail vein. Blood droplet was applied onto a test strip that was inserted into a glucose analyzer (glucometer, Accu-Chek Performa). Rats were then injected intraperitoneally with 1 g of glucose per kg fasting body mass, and blood glucose levels were determined 15, 30, 60, 90 and 120 min after glucose. The sampling and measurement were conducted at the age of 18 weeks.

### Results:

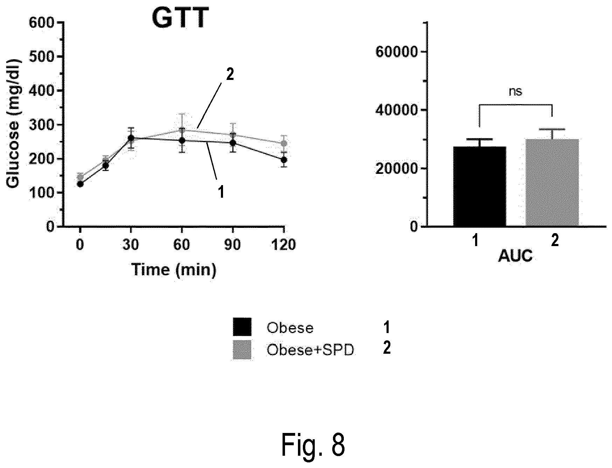

10 weeks of spermidine (Obese + SPD) treatment did not change the glucose clearance curve (represented as AUC as well) compared to control obese rats (Obese) (see Fig. 8).

### Summary:

Despite the beneficial cardiovascular effects of nicotinamide, it causes impaired glucose homeostasis, leading to several side effects, as e.g. an increase in fasting glucose and in fasting insulin and to impaired glucose homeostasis. As shown, these negative side effects of nicotinamide can be compensated by spermidine.

As widely clinically-used statins also cause a similar side effect on glucose tolerance, it is possible that spermidine could be combined also with statins to mitigate serious adverse effects of statin application, including new-onset diabetes mellitus, myopathy and haemorrhagic stroke.

## Claims

1. A composition comprising spermidine and/or a pharmaceutically acceptable salt thereof for use in the prevention and/or alleviation of adverse effects on the glucose and/or insulin homeostasis caused by nicotinamide and/or a pharmaceutically acceptable salt thereof.

2. The composition for the use according to claim 1, wherein the adverse effects on the glucose and/or insulin homeostasis are selected from the group consisting of an increase in blood glucose level, diabetes type 2, and an increase in blood insulin level.

3. The composition for the use according to claim 1 or 2, wherein spermidine and/or a pharmaceutically acceptable salt thereof and nicotinamide and/or a pharmaceutically acceptable salt thereof are administered in a molar ratio ranging from 1:5 to 1:100, preferably 1:5 to 1:75, more preferably 1:5 to 1:50, more preferably from 1:8 to 1:25, more preferably 1:10 to 1:15.

4. The composition for the use according to any one of claims 1 to 3, wherein spermidine and/or a pharmaceutically acceptable salt thereof is provided as an extract, preferably as a plant extract, more preferably as a wheat germ extract or a soy bean extract.

5. The composition for the use according to any one of claims 1 to 4, wherein the composition is administered before, together or consecutively to nicotinamide and/or a pharmaceutically acceptable salt thereof.

6. The composition for the use according to any one of claims 1 to 5, wherein the composition and/or nicotinamideand/or a pharmaceutically acceptable salt thereof is administered orally or intravenously.

## Patentansprüche

1. Zusammensetzung, umfassend Spermidin und/oder ein pharmazeutisch annehmbares Salz davon zur Verwendung bei der Vorbeugung und/oder Linderung von Nebenwirkungen auf die Glukose- und/oder Insulinhomöostase, verursacht durch Nicotinamid und/oder ein pharmazeutisch annehmbares Salz davon.

2. Zusammensetzung zur Verwendung nach Anspruch 1, wobei die Nebenwirkungen auf die Glukose- und/oder Insulinhomöostase aus der Gruppe ausgewählt sind, die aus einem Anstieg des Blutglukosespiegels, Diabetes Typ 2 und einem Anstieg des Blutinsulinspiegels besteht.

3. Zusammensetzung zur Verwendung nach Anspruch 1 oder 2, wobei Spermidin und/oder ein pharmazeutisch annehmbares Salz davon und Nikotinamid und/oder ein pharmazeutisch annehmbares Salz davon in einem molaren Verhältnis im Bereich von 1:5 bis 1:100, vorzugsweise 1:5 bis 1:75, noch bevorzugter 1:5 bis 1:50, noch bevorzugter 1:8 bis 1:25, noch bevorzugter 1:10 bis 1:15 verabreicht werden.

4. Zusammensetzung zur Verwendung nach einem der Ansprüche 1 bis 3, wobei Spermidin und/oder ein pharmazeutisch annehmbares Salz davon als Extrakt, vorzugsweise als Pflanzenextrakt, besonders bevorzugt Weizenkeimextrakt oder Sojabohnenextrakt, bereitgestellt wird.

5. Zusammensetzung zur Verwendung nach einem der Ansprüche 1 bis 4, wobei die Zusammensetzung vor, zusammen oder nacheinander mit Nicotinamid und/oder einem pharmazeutisch akzeptablen Salz davon verabreicht wird.

6. Zusammensetzung zur Verwendung nach einem der Ansprüche 1 bis 5, wobei die Zusammensetzung und/oder Nicotinamid und/oder ein pharmazeutisch annehmbares Salz davon oral oder intravenös verabreicht wird.

## Revendications

1. Composition comprenant de la spermidine et/ou un sel pharmaceutiquement acceptable de celle-ci, destinée à être utilisée dans la prévention et/ou l'atténuation des effets indésirables sur l'homéostasie du glucose et/ou de l'insuline causés par la nicotinamide et/ou un sel pharmaceutiquement acceptable de celle-ci.

2. Composition destinée à être utilisée selon la revendication 1, dans laquelle les effets indésirables sur l'homéostasie du glucose et/ou de l'insuline sont choisis dans le groupe constitué par une augmentation du taux de glucose sanguin, le diabète de type 2 et une augmentation du niveau d'insuline sanguin.

3. Composition destinée à être utilisée selon la revendication 1 ou 2, dans laquelle la spermidine et/ou un sel pharmaceutiquement acceptable de celle-ci et la nicotinamide et/ou un sel pharmaceutiquement acceptable de celle-ci sont administrés dans un rapport molaire allant de 1:5 à 1:100, de préférence de 1:5 à 1:75, plus préférablement de 1:5 à 1:50, plus préférablement de 1:8 à 1:25, plus préférablement de 1:10 à 1:15.

4. Composition destinée à être utilisée selon l'une quelconque des revendications 1 à 3, dans laquelle la spermidine et/ou un sel pharmaceutiquement acceptable de celle-ci sont fournis sous forme d'extrait, de préférence sous forme d'extrait végétal, plus préférablement sous forme d'extrait de germe de blé ou d'extrait de soja.

5. Composition destinée à être utilisée selon l'une quelconque des revendications 1 à 4, dans laquelle la composition est administrée avant, conjointement avec ou consécutivement à la nicotinamide et/ou à un sel pharmaceutiquement acceptable de celle-ci.

6. Composition destinée à être utilisée selon l'une quelconque des revendications 1 à 5, dans laquelle la composition et/ou la nicotinamide et/ou un sel pharmaceutiquement acceptable de celle-ci sont administrés par voie orale ou intraveineuse.
